(19) Europäisches Patentamt
European Patent Office
Office européen des brevets

(11) EP 0 677 521 B1

(12) **EUROPÄISCHE PATENTSCHRIFT**

(45) Veröffentlichungstag und Bekanntmachung des Hinweises auf die Patenterteilung:
**09.08.2000 Patentblatt 2000/32**

(51) Int. Cl.$^7$: **C07D 311/94**, C07C 33/34, C11B 9/00

(21) Anmeldenummer: **95102462.9**

(22) Anmeldetag: **22.02.1995**

# (54) Indan als Riechstoff

Indane as perfume

Indane utile comme parfum

(84) Benannte Vertragsstaaten:
**CH DE ES FR GB IT LI NL**

(30) Priorität: **10.03.1994 CH 70894**

(43) Veröffentlichungstag der Anmeldung:
**18.10.1995 Patentblatt 1995/42**

(73) Patentinhaber:
**GIVAUDAN-ROURE (INTERNATIONAL) S.A.**
**CH-1214 Vernier (CH)**

(72) Erfinder:
- **Frater, Georg**
  **CH-8404 Winterthur (CH)**
- **Müller, Urs**
  **CH-8051 Zürich (CH)**
- **Petrzilka, Martin**
  **CH-8620 Wetzikon (CH)**

(74) Vertreter: **Buntz, Gerhard et al**
**GIVAUDAN-ROURE (INTERNATIONAL) SA,**
**Postfach 3255**
**4002 Basel (CH)**

(56) Entgegenhaltungen:
**DE-A- 2 440 526**
**US-A- 3 660 311**
**US-A- 4 162 256**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7 (HRS)/3.6

## Beschreibung

Die Erfindung betrifft einen neuen Riechstoff, nämlich das 7R / 7S-Diastereoisomerengemisch der Formel

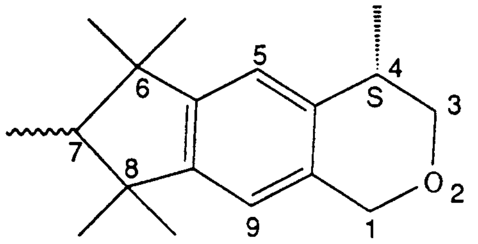

I

in reiner Form.

Die Erfindung betrifft auch Riechstoffkompositionen mit einem Gehalt an I, sowie die Verwendung von I als Riechstoff.

Das Gemisch I ist gemäss folgendem Schema zugänglich:

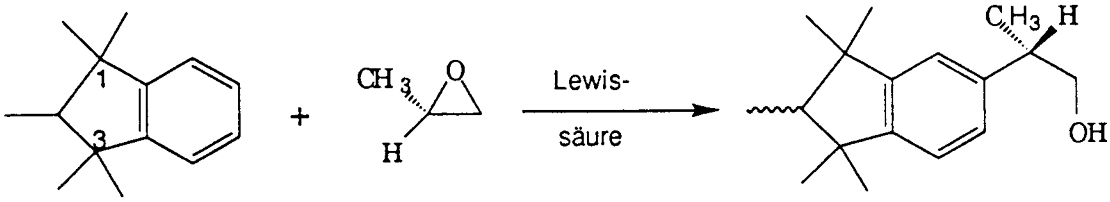


III IV II

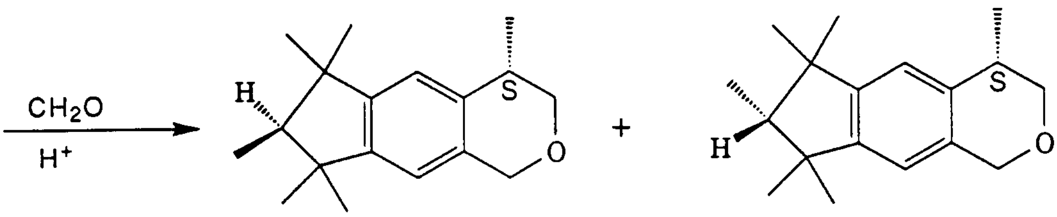


(a) I (b)

Der Isochromanringschluss von II wird zweckmässigerweise in an sich bekannter Weise, also z.B. mittels Formaldehyd, oder einem synthetischen Analogon davon, z.B. dem Acetal, insbesondere dem Dimethyl- oder Diäthylacetal oder auch Dihexylacetal, bewerkstelligt.

Die Temperatur liegt zweckmässigerweise zwischen ca. 0°C und ca. 200° C., vorzugsweise zwischen ca. 85° C und ca. 150° C.

Katalysator: eine Protonensäure, beispielsweise p-Toluolsulfonsäure oder Phosphorsäure, etc.

Säurekonzentration: 1 bis 100% (G/G) der Menge II.
Verhältnis II: $CH_2O$: ca. 0,1 - ca. 1.

Das Acetal kann auch aus $CH_2O$ und einem niederen Alkanol in situ hergestellt werden.

Auch für die Friedel-Crafts-reaktion von III zu II gelten die an sich gut bekannten Bedingungen, also beispielsweise:

Lewissäure:
insbesondere $AlCl_3$, aber auch $SnCl_4$, $SnBr_4$, $TiCl_4$, $BCl_3$, etc.
Temperaturbereich:
ca. -30° C bis ca. +30° C, insbesondere
ca. -20° C bis ca. -0° C.
Lösungsmittel:
gesättigter aliphatischer, gegebenenfalls halogenierter Kohlenwasserstoff, beispielsweise Pentan, oder Hexan, etc., aber auch die üblichen, gegebenenfalls halogenierten Aromaten, oder auch $CS_2$.
Verhältnis Lösungsmittel zu III:
ca. 1:10 bis ca. 10:1.
Verhältnis Katalysator: IV:
ca. 1:1 bis ca. 2:1.
Verhältnis III : IV
ca. 1:1 bis ca. 10:1.
Aufarbeitung:
Zugabe eines Alkanols oder aliphatischen Aethers zum Reaktionsgemisch, oder auch Gemische solcher Lösungsmittel, etc.

Das Diastereoisomerengemisch I weist ausgesprochen überraschende Eigenschaften auf.
Verglichen mit dem handelsüblichen Gemisch der beiden Racemate der Formel

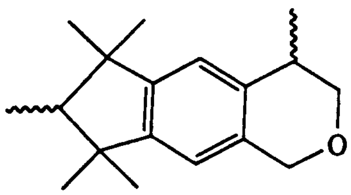

I'

siehe S. Arctander, Perfume and Flavor Chemicals I, Montclair, N.J. 1969, item 1581
ist seine Verwendung ausgesprochen bevorzugt, was sich sofort aus der folgenden Gegenüberstellung aller entsprechender Geruchsschwellenwerte ergibt:

| | | | |
|---|---|---|---|
| (4S)-cis-Isomeres | 0,3 ng/l Luft | I (b) | I' |
| (4S)-trans-Isomeres | 0,6 ng/l Luft | I (a) | I' |
| (4R)-cis-Isomeres | 200 ng/l Luft | | I' |
| (4R)-trans-Isomeres | 400 ng/l Luft. | | I' |

Durch die Verwendung von I wird also in jedem Fall Einschleppen, Mitschleppen von ca. 50%, unnötigem, vollkommen funktionslosem Ballast vermieden. Der Begriff "Gemisch I in reiner Form" dient also im vorliegenden Fall zur Abgrenzung dieses neuen Gemisches I vom Handelsprodukt der obigen Formel I', beinhaltet also ein Gemisch, das im wesentlichen frei von den (4R)-Isomeren ist.
Im übrigen kann das Gemisch I als Riechstoff genau gleich wie der bekannte Isochromanmoschuskörper, das Indan I' verwendet werden, wobei jedoch, entsprechend den obigen Geruchs-schwellenwerten, geringere Konzentrationen erforderlich sind.

<u>Beispiel</u>

a) Zu einer auf - 10° C gekühlten Lösung von 26,1 g 1,1,2,3,3-Pentamethylindan in 15 ml $CS_2$ werden 7,5 g $AlCl_3$ gegeben. Zum gelb-braunen Gemisch gibt man nun langsam bei -5° C bis -3° C 2,4 g S (-)-Propylenoxyd, gelöst in

2,5 ml $CS_2$. Nach Beendigung der Zugabe wird das Gemisch 30 Minuten bei 0° C weiter gerührt und dann auf Eis-Wasser gegeben. Die Aufarbeitung erfolgt, indem man in ein Gemisch von Hexan-tert.butyl-methyläther im Verhältnis 1:1 aufnimmt.

Das rohe Produkt wird zuerst bei 180-200° C destilliert und zwar bei 0,1 mm Hg, und dann durch Kristallisation weiter gereinigt, und zwar durch Kristallisation aus Hexan.

Der Schmelzpunkt des Produktes (2S, 2'R,S)-2-1',1',2',3',3'-Pentamethylindan-5'-yl-propan-1-ol beträgt 73 - 74° C, $[\alpha]_D^{22}$ ($C_2H_5OH$, c = 1,0) -6.23° .

b) Das unter a) erhaltene 2-Indan-5'-yl-propan-1-ol wird mit Paraformaldehyd, 1-Hexanol und Phosphorsäure genau nach der Vorschrift des Beispiels 2 der US-Patentschrift 3 910 984 behandelt, und das Produkt, nämlich (4S, 7RS) - 1,3,4,6,7,8-Hexahydro-4,6,6,7,8,8-hexamethylcyclopenta [g]-2-benzopyran, in guter Ausbeute erhalten. $[\alpha]_D^{22}$ ($C_2H_5OH$, c = 1.18) +21,4° .

**Patentansprüche**

**1.** Das 7R / 7S-Diastereoisomerengemisch der Formel

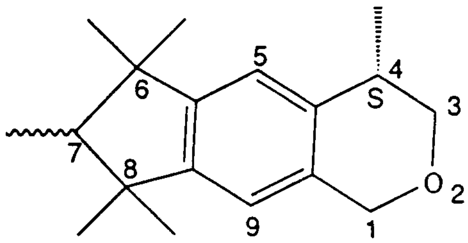

I

in reiner Form.

**2.** Das 2R/2S-Diastereoisomerengemisch der Formel

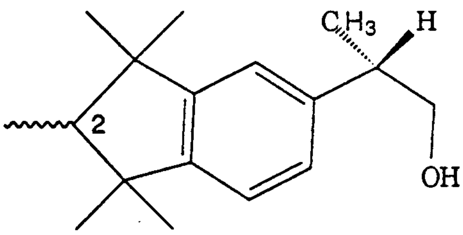

II

in reiner Form.

**3.** Riechstoffkomposition, gekennzeichnet durch einen Gehalt an Diastereoisomerengemisch der Formel I gemäss Anspruch 1, wobei das Gemisch im wesentlichen frei von (4R)-Isomeren ist.

**4.** Verfahren zur Herstellung des Diastereoisomerengemisches der Formel I gemäss Anspruch 1, dadurch gekennzeichnet, dass man das 2R/2S-Diastereoisomerengemisch der Formel

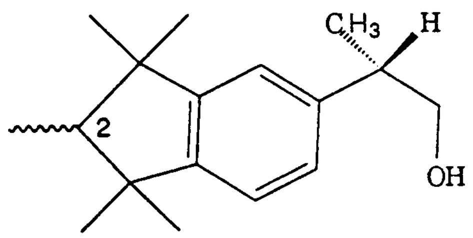

II

einem Isochromanringschluss unterwirft.

5. Verwendung des Diastereoisomerengemisches der Formel I gemäss Anspruch 1 als Riechstoff.

**Claims**

1. The 7R/7S-diastereoisomer mixture of the formula

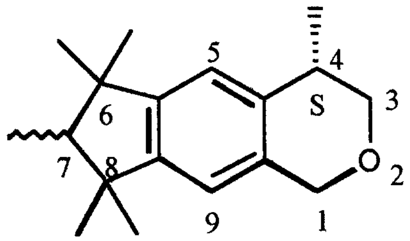

I

in pure form.

2. The 2R/2S-diastereoisomer mixture of the formula

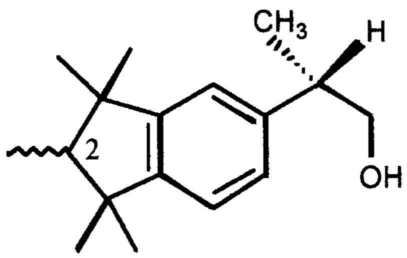

II

in pure form.

3. An odorant composition, characterized by a content of diastereoisomer mixture of formula I according to claim 1, with the mixture being essentially free from (4R)-isomers.

4. A process for the manufacture of the diastereoisomer mixture of formula I according to claim 1, characterized by subjecting the 2R/2S-diastereoisomer mixture of the formula

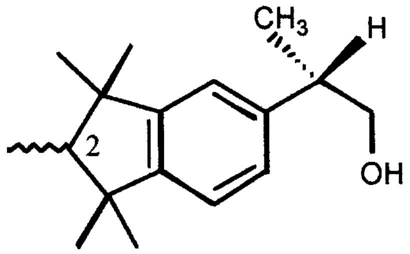

II

to an isochromane ring closure.

5. The use of the diastereoisomer mixture of formula I according to claim 1 as an odorant.

**Revendications**

1. Mélange de diastéréoisomères 7R/7S de formule

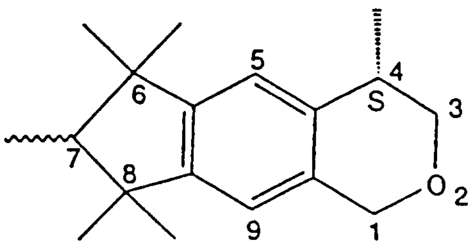

I

sous forme pure.

2. Mélange de diastéroisomères 2R/2S de formule

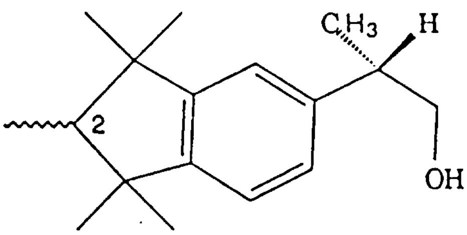

II

sous forme pure.

3. Composition de produit odorant, caractérisée en ce qu'elle contient un mélange de diastéroisomères de formule I selon la revendication 1, le mélange étant pour l'essentiel exempt des isomères (4R).

4. Procédé de préparation du mélange de diastéroisomères de formule I selon la revendication 1, caractérisé en ce qu'on soumet à une cyclisation de l'isochromanne le mélange de diastéréoisomères 2R/2S de formule

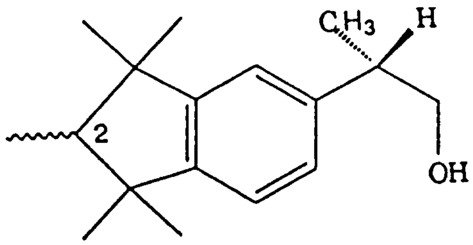

II

**5.** Utilisation, en tant que produit odorant, du mélange de diastéréolsomères de formule I selon la revendication 1.